# EUROPÄISCHE PATENTSCHRIFT

(11) **EP 0 575 916 B1**
(45) Veröffentlichungstag und Bekanntmachung des Hinweises auf die Patenterteilung: **26.01.2000**
(21) Anmeldenummer: 93109795.0
(22) Anmeldetag: 18.06.1993
(51) Int. Cl.: A61B 5/15

(54) **Blutentnahme-Vorrichtung**
Blood sampling device
Dispositif de prise de sang

(30) Priorität: 22.06.1992 DE 4220309
(43) Veröffentlichungstag der Anmeldung: 29.12.1993
(73) Patentinhaber: pvb medizintechnik gmbh & co. kg, 85614 Kirchseeon/Eglharting (DE)
(72) Erfinder: Von Berg, Peter, Tiburon, CA 94920 (US)
(74) Vertreter: von Bülow, Tam, Dr.

(56) Entgegenhaltungen:
- EP-A- 0 102 073
- EP-A- 0 301 913
- EP-A- 0 376 603
- WO-A-89/01616
- US-A- 4 673 386

## Beschreibung

Die Erfindung bezieht sich auf eine Blutentnahme-Vorrichtung. Eine Vorrichtung gemäß dem Oberbegriff des Patentanspruches 1 ist aus dem Dokument WO-A-89/01616 bekannt. Weitere Vorrichtungen werden in den Dokumenten EP-A2-0 302 752 und WO 88/01846 beschrieben. In Fällen, bei denen ein Infusionsschlauch oder ein Katheter in eine Vene oder Arterie eines Patienten eingesetzt ist, beispielsweise für eine Tropfinfusion und/oder eine permanente, direkte Blutdruckmessung wird, wenn für Untersuchungszwecke eine Blutprobe entnommen werden soll, diese an einer von außen zugänglichen Stelle des Infusionsschlauches entnommen. Hierzu sehen die oben genannte Vorrichtungen Entnahmestellen vor, die mit einem Strömungskanal des Infusionssystems in Verbindung stehen und durch eine Kolbenspritze von außen zugänglich sind.

Bevor eine Blutentnahme aus diesem System vorgenommen werden kann, muß zunächst dafür gesorgt werden, daß sich im Bereich der Entnahmestelle nur unverdünntes Blut des Patienten und keine Anteile von Infusionslösungen oder Verdünnungsmitteln befinden, die bei der direkten Blutdruckmessung zum Verhindern der Koagulation des Blutes zugeführt werden. Zu diesem Zwecke sieht die WO 88/01846 zwei Entnahmestellen vor. Die näher zum Patienten liegende (vordere) Entnahmestelle dient der eigentlichen Entnahme der Blutprobe. Die weiter vom Patienten entfernt liegende (hintere) Entnahmestelle dient dazu, vorübergehend Infusionslösungen aus dem System zu entfernen, damit an der vorderen Entnahmestelle nur noch unverdünntes Blut vorhanden ist. Nach der Blutentnahme an der vorderen Entnahmestelle wird dann die an der hinteren Entnahmestelle entnommene Menge aus Infusionslösung und Blut wieder dem System zugeführt.

Bei der WO 88/01846 erfolgt die Entnahme und das Wiederzuführen an der hinteren Entnahmestelle mittels einer herkömmlichen Spritze, deren Nadel einen ansonsten dichten Verschlußstopfen durchdringt. Dies setzt aber ein aufwendiges Hantieren voraus, bringt die Gefahr mit sich, daß durch die Spritzennadel Verunreinigungen, Keime und sonstige Krankheitserreger dem System zugeführt werden und bringt für das Klinikpersonal die Gefahr mit sich, daß es sich beim Entfernen der Nadel daran verletzt und infiziert, was in der Vergangenheit schon zu einer großen Anzahl von Aids-Infektionen bei Klinikpersonal geführt hat.

Es wurde daher schon vorgeschlagen, insoweit ein geschlossenes System zu verwenden, als die hintere Entnahmestelle als ein permanent in das Infusions- oder Kathetersystem integrierter Zwischenspeicher mit einer Kolben-/Zylinderanordnung ausgestaltet ist, die von außen nicht mehr zugänglich ist (vgl. Firmenprospekt der Firma Baxter mit dem Titel "New Vamp; a closed system for easier, safer blood sampling from invasive lines"; sowie Firmenprospekt der Firma Pfrimmer Viggo GmbH & Co. KG mit dem Titel "Saw draw; geschlossenes Blutentnahmesystem mit Statham-Einmal-Druckwandler DTX/plus).

Bei diesen bekannten Blutentnahmevorrichtungen ist jedoch, insbesondere wenn mehrfach nacheinander Blut von einem Patienten entnommen werden soll, die Sterilität des Zwischenspeichers nicht immer gewährleistet. Wird beispielsweise bei der WO 88/01846 als Zwischenspeicher eine herkömmliche Kolbenspritze verwendet, so können durch Herausziehen und anschließendes Wiedereinschieben der Kolbenstange in den Zylinderraum der Spritze Bakterien und Keime eingeschleppt werden.

Aufgabe der Erfindung ist es daher, die bekannten Blutentnahmevorrichtungen dahingehend zu verbessern, daß der bei der Blutentnahme als Reservoir für zwischengespeicherte Mengen von mit Infusionslösungen vermischtem Blut verwendete Zwischenspeicher absolut steril ist und zwar auch bei mehrmaligem Gebrauch.

Dieser Aufgabe wird durch die im Kennzeichenteil des Patentanspruches 1 angegebenen Merkmale gelöst. Vorteilhafte Ausgestaltung und Weiterbildung der Erfindung sind den Unteransprüchen zu entnehmen.

Bei der Erfindung ist der Zwischenspeicher gegenüber der Außenwelt absolut bakteriendicht. Bei der Betätigung gelangen keine bewegten Teile nach außen, die später wieder Bakterien oder Keime einschleppen können. Gleichwohl ist ein Luftaustausch, der für die Verschiebung des Kolbens erforderlich ist, gewährleistet. Auch ist die Vorrichtung nach der Erfindung einfach zu montieren und aus wenigen Spritzgußteilen herzustellen.

Ein weiterer Vorteil der Erfindung ist die Bedienung der Vorrichtung durch Drehen des Deckels, was die Handhabung erleichtert. Insbesondere im Zusammenhang mit den Drehbegrenzungsanschlägen wird hierdurch sichergestellt, daß der Kolben in der einen Grenzstellung wirklich ganz nach unten gefahren ist, so daß der Zylinderraum dann vollständig entleert ist. Vorzugsweise ist die Dichtung zwischen Gehäuse und Deckel als Ringdichtung ausgeführt, für die beispielsweise ein O-Ring verwendet werden kann. Diese Dichtung ist leicht zu montieren und erfüllt ohne großen technischen Aufwand ihren Zweck.

Nach einer vorteilhaften Weiterbildung der Erfindung ist der Deckel mit einem Schnappverschluß gegenüber dem Gehäuse in Axialrichtung gesichert. Damit ist ein unfreiwilliges Öffnen des Gehäuses unmöglich, wodurch jegliche Kontamination vermieden wird.

Ein weiterer wesentlicher Vorteil der Erfindung liegt darin, daß der Kolben einschließlich Kolbenstange nur innerhalb des Gehäuses bewegt wird und nicht mit irgendwelchen Abschnitten nach außen tritt. Dies wird dadurch erreicht, daß die Länge der Gewindehülse mindestens der Verschiebelänge des Kolbens entspricht.

Im folgenden wird die Erfindung anhand eines Ausführungsbeispieles im Zusammenhang mit der Zeichnung ausführlicher erläutert. Es zeigt:
- Fig. 1: einen Querschnitt der Blutentnahmevorrichtung;
- Fig. 2: einen um 90 Grad gegenüber der Darstellung der Fig. 1 gedrehten Querschnitt der Blutentnahmevorrichtung;
- Fig. 3 und 4: Querschnitte des Gehäuses der Blutentnahmevorrichtung entsprechend den Fig. 1 bzw. 2;
- Fig. 5: eine Draufsicht auf die Öffnungsseite des Gehäuses der Fig. 3;
- Fig. 6: eine Seitenansicht des bei der Erfindung verwendeten Kolbens;
- Fig. 7: eine Draufsicht des Kolbens in Sicht auf die Kolbenstange;
- Fig. 8: eine Draufsicht eines bei der Erfindung verwendeten Verdrehschutzes;
- Fig. 9: eine Draufsicht auf die Öffnungsseite des Gehäusedeckels;
- Fig. 10 und 11: Querschnitte des Gehäusedeckels, entsprechend den Ansichten der Fig. 1 und 2; und
- Fig. 12: eine Draufsicht der Außenseite des Gehäusedeckels.

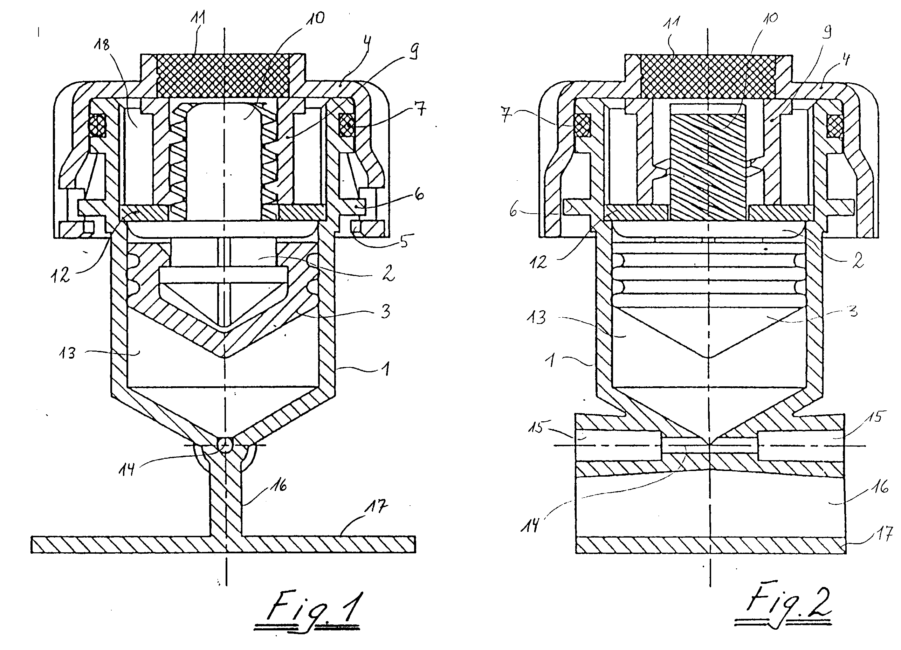

Die Fig. 1 und 2 zeigen die Vorrichtung im zusammengebauten Zustand. Die Vorrichtung besitzt ein Gehäuse 1, in dessen Inneren ein Kolben 2 verschieblich geführt ist. Die Spitze des Kolbens 2 ist von einer Kolbendichtung 3 aus flexiblem Material umhüllt und dichtet den Kolben gegenüber der Innenwandung des Gehäuses 1 ab. Die nach oben hin (bezogen auf die Darstellung der Fig. 1 und 2) offene Seite des Gehäuses 1 ist durch einen Deckel 4 verschlossen, der die Öffnungsseite des Gehäuses kappenartig übergreift und der durch Rastvorsprünge 5, die eine ringsumlaufende Schulter 6 übergreifen, an dem Gehäuse verriegelbar ist, wobei sich der Deckel 4 gegenüber dem Gehäuse 1 drehen läßt.

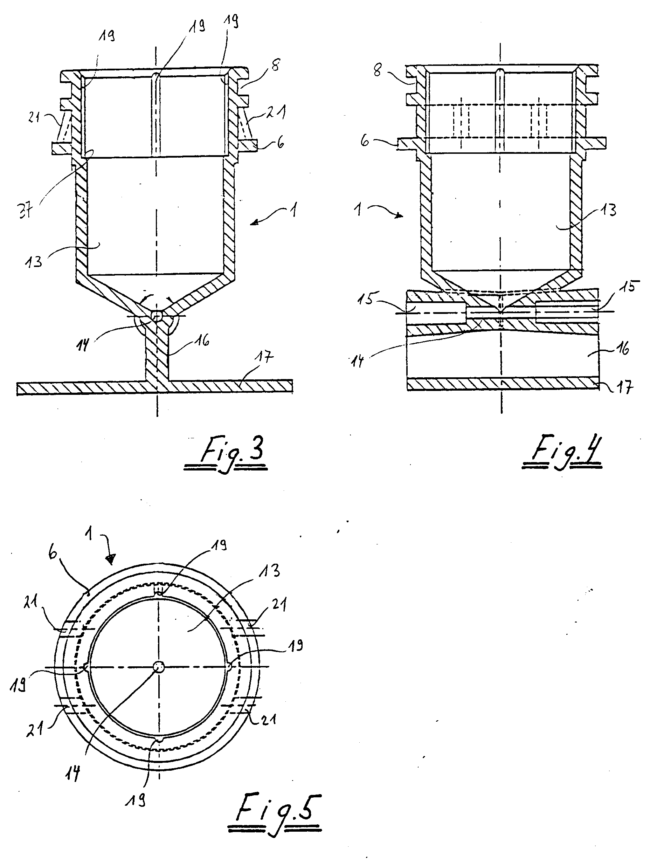

Zwischen dem Gehäuse 1 und dem Deckel 4 ist eine Dichtung 7 vorhanden, die hier als O-Ring ausgebildet ist. Diese Dichtung ist in einer Nut 8 (Fig. 3) an der Außenseite des Gehäuses gehalten.

Der Deckel 4 weist eine sich axial erstreckende Gewindehülse 9 auf, die ein Innengewinde mit relativ großer Steigung besitzt. Dieses Innengewinde wirkt mit einem Außengewinde an einer Kolbenstange 10 des Kolbens 2 zusammen derart, daß bei Drehen des Deckels 4 relativ zum Gehäuse 1 der Kolben in Axialrichtung verschoben wird.

Der Deckel 4 weist an seiner Stirnseite eine Öffnung auf, in die ein Sterilfilter 11 eingesetzt ist. Dieses Filter ist luftdurchlässig, aber dicht gegen Bakterien, Keime und sonstige Verunreinigungen.

Damit beim Drehen des Deckels 4 der Kolben 2 nur linear verschoben wird, sich jedoch nicht dreht, ist ein Verdrehschutz 12 vorgesehen.

Durch die Kolbendichtung 3 wird der Innenraum des Gehäuses 1 in zwei Kammern unterteilt, nämlich eine mit einem Durchflußkanal 14 unmittelbar in Verbindung stehende Kammer 13 und eine obere, sich in Richtung zur Öffnung des Gehäuses 4 erstreckende Entlüftungskammer 18. Diese Entlüftungskammer 18 ist auch bei Verschieben des Kolbens 2 gegenüber der Umgebung abgedichtet, und zwar zum einen über die Dichtung 7 und zum anderen über das Sterilfilter 11, so daß keinerlei Bakterien, Keime oder sonstige Verunreinigungen in das Innere des Gehäuses 1 gelangen können.

Im Bereich des Durchflußkanales 14 geht das Gehäuse von der zylindrischen Form mit Kegelstumpfspitze in einen achsparallel verlaufenden Steg 16 über und endet in einem quer dazu verlaufenden zweiten Steg 17.

Wie aus Fig. 2 zu erkennen ist, verbreitern sich die beiden Enden des Durchflußkanales 14 zu Anschlüssen 15, an die Schläuche angeschlossen werden können.

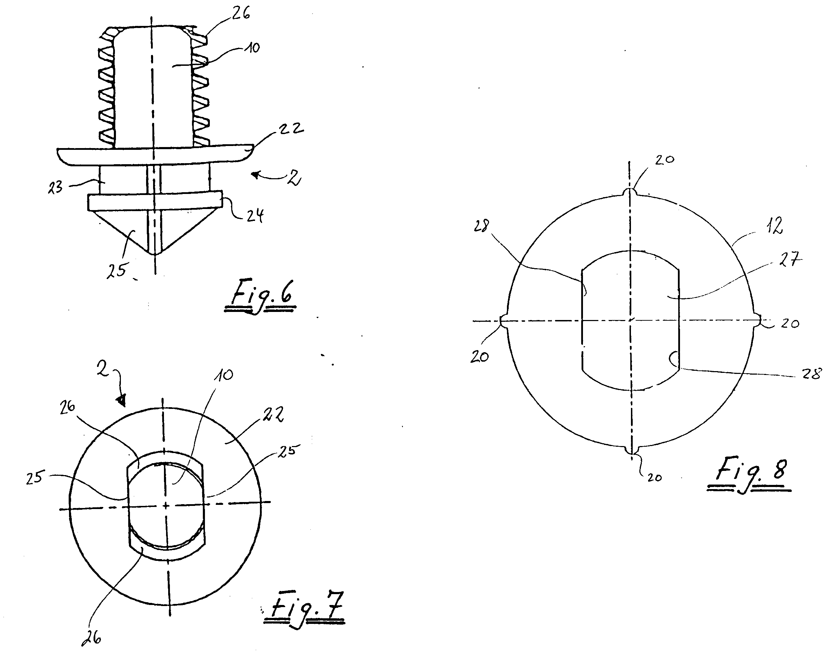

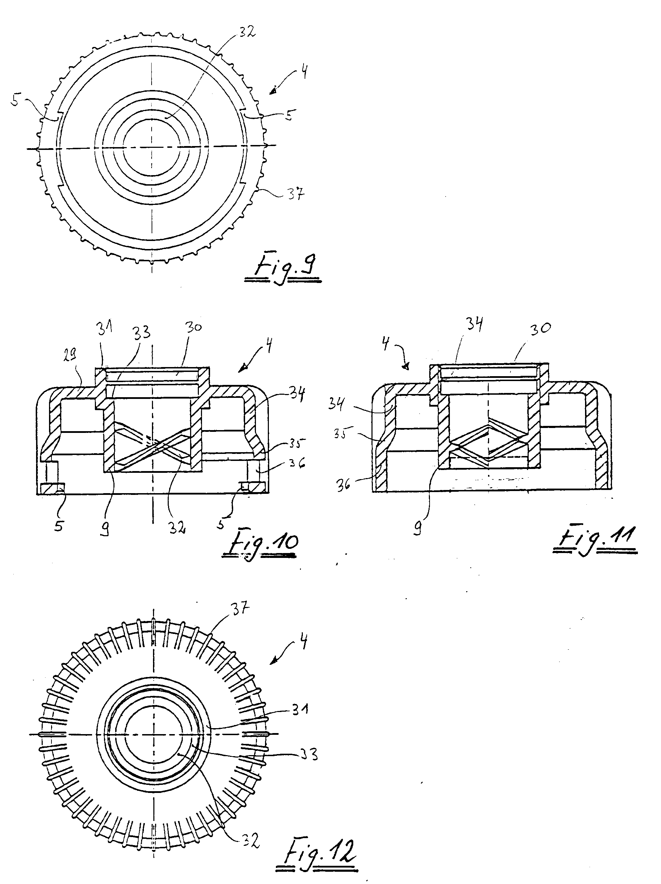

Aus den Fig. 3 bis 5 sind weitere Einzelheiten des Gehäuses 1 zu erkennen. In dem zum Deckel weisenden Bereich sind in der Innenwandung des Zylinderraumes 13 vier axial verlaufende Nuten 19 vorgesehen, die zur Führung der Verdrehsicherung 12 dienen, die mit vier entsprechenden Vorsprüngen 20 (Fig. 8) in diese Nuten eingreift. Die Nuten 19 enden an einer Schulter 37, die als axialer Anschlag für den Verdrehschutz 12 dient.

Weiter ist darauf hinzuweisen, daß das dem Durchflußkanal 14 zugewandte, kegelstumpfförmige Ende des Zylinderraumes 13 unmittelbar in den Durchflußkanal 14 mündet, d.h. ein in diesen Raum entsprechend eingesetzter Kegelstumpf würde mit seiner Spitze in den Durchflußkanal 14 hineinragen. Da im Ausführungsbeispiel der Fig. 1 und 2 die Kolbendichtung eine entsprechende Form hat, ist sichergestellt, daß bei vollständig niedergedrücktem Kolben keinerlei Flüssigkeitsreste im Zylinderraum 13 verbleiben.

Anschließend an die Schulter 6 erstrecken sich vier Verstärkungsrippen 21 bis in die Nähe der Ringnut 8. Diese Verstärkungsrippen können auch in Verbindung mit einem nicht dargestellten Vorsprung des Deckels 4 als Drehbegrenzungsanschlag dienen. Zusätzlich kann die der Öffnung des Zylinderraumes 13 abgewandte Seite der Schulter 6 einen oder mehrere Drehbegrenzungsanschläge mit einer Auflaufschräge und einer daran anschließenden Vertiefung aufweisen, in die die Vorsprünge 5 in den Grenzstellungen einrasten.

Die Fig. 6 und 7 zeigen detaillierter den Kolben 2, dessen Kolbenstange 10 mit einem Außengewinde 26 versehen ist. An dieses Außengewinde 26 schließt sich eine radial vorspringende Schulter 22 an, deren Durchmesser - mit gewissem Spiel - dem Innendurchmesser des Zylinderraumes 13 entspricht. An diese Schulter schließt sich ein Abschnitt geringeren Durchmessers 23 an, der auch nur aus zwei sich kreuzenden Stegen bestehen kann. Daran schließt sich wiederum eine dem gegenüber radial vorstehende Schulter 24 an und daran schließlich ein "konischer" Abschnitt 25, der wiederum nur aus zwei sich kreuzenden Stegen bestehen kann. Die Kolbendichtung 3 ist zwischen den beiden Schulter 22 und 24 in beiden Axialrichtungen gehalten und liegt mit ihrer Innenwandung an dem Rand der Schulter 24 an. Der konische Abschnitt 25 ragt in einen Hohlraum der Kolbendichtung 3 hinein, wobei in niedergedrücktem Zustand des Kolbens 2 die Spitze des konischen Abschnittes 24 die Innenwandung der entsprechend konischen Spitze der Kolbendichtung 3 berührt und diese damit in den Durchflußkanal 14 hineindrückt.

Wie aus Fig. 7 zu erkennen ist, hat die Kolbenstange 10 zwei abgeflachte Seiten 25, so daß ihr Gewinde 26 nicht vollständig um den Außenumfang der Kolbenstange herum verläuft. Die Verdrehsicherung 12 (Fig. 8), die als im wesentlichen ebene Scheibe ausgebildet ist, weist eine mittige Öffnung 27 auf, die der Kontur der Kolbenstange 10 angepaßt ist und also ebenfalls zwei einander gegenüberliegende, abgeflachte Seiten 28 aufweist. Damit kann sich der Kolben 2 relativ zur Verdrehsicherung 12 nicht drehen. Da die Verdrehsicherung 12 ihrerseits durch die Vorsprünge 20 in den Nuten 19 des Gehäuses 1 verdrehsicher gehalten ist, kann der Kolben 2 also nur Bewegungen in Axialrichtung ausführen, wenn der Deckel 4 gedreht wird.

Der Deckel 4 wird im Zusammenhang mit den Fig. 9 bis 12 beschrieben.

Die nach außen weisende im wesentlichen ebene Wand 29 des Deckels 4 weist eine mittige Öffnung 30 auf, in die der Bakterienfilter 11 (Fig. 1) eingesetzt ist. An diese Öffnung schließt sich ein von der Wand 29 abstehender zylindrischer Abschnitt 31 an, der der Halterung und Führung des Bakterienfilters 11 dient. Ausgehend von der Innenseite der Wand 19 schließt sich an den zylindrischen Abschnitt 31 die Gewindehülse 9 an, die ein Innengewinde 32 besitzt, das mit dem Außengewinde 26 der Kolbenstange 10 zusammenwirkt. Der Innendurchmesser der Gewindehülse 9 ist geringer als der Durchmesser der Öffnung 30, so daß eine durch diese Durchmesser-Verkleinerung gebildete Stufe 33 als Abstützung für den Bakterienfilter 11 dient. In der Öffnung 30 ist eine radial nach innen ragende Rippe 34 vorgesehen, die als zusätzliche Sicherung gegen Herausfallen des Bakterienfilters 11 dient.

Anschließend an den äußeren Umfang der Wand 29 folgt ein zylindrischer Abschnitt 34, dessen Durchmesser dem Durchmesser der die Nut 8 des Gehäuses 1 begrenzenden Seitenwände entspricht, so daß dieser Abschnitt 34 in Zusammenwirken mit dem Dichtungsring 7 (Fig. 1 und 2) die Dichtungsfunktion übernimmt.

Anschließend an den zylindrischen Abschnitt 34 folgt ein sich verbreiternder Abschnitt 35, der in einen zylindrischen Abschnitt 36 übergeht. Der Innendurchmesser des zylindrischen Abschnittes 36 ist etwas größer als der Außendurchmesser der Schulter 6.

In dem zylindrischen Abschnitt 36 sind die radial nach innen ragenden Rastvorsprünge 5 ausgebildet, die die Schulter 6 hintergreifen und den Deckel gegenüber dem Gehäuse verriegelt. Diese Rastvorsprünge 5 dienen auch als Drehbegrenzungsanschlag, wie oben im Zusammenhang mit dem Gehäuse beschrieben.

Am Außenumfang des Deckels 4 befindet sich eine Rändelung 37, die ein Ergreifen und Drehen des Deckels von Hand erleichtert.

## Patentansprüche

1. Blutentnahme-Vorrichtung mit einer als Zwischenspeicher für verdünntes Blut dienenden Kolben-/Zylinderanordnung mit einem im wesentlichen zylindrischen Gehäuse (1), das mit einem Durchflußkanal (14) in Verbindung steht und durch einen drehbaren, kappenartigen Deckel (4) verschlossen ist und einem im Innern des Gehäuses (1) verschieblichen Kolben (2), der über ein Gewinde (26) mit dem Deckel (4) in Verbindung steht, dadurch gekennzeichnet, daß der Deckel (4) eine Gewindehülse (9) aufweist, die mit dem Gewinde (26) der Kolbenstange (10) des Kolbens (2) zusammenwirkt, der Deckel (4) eine Dichtung (7) enthält, die das Gehäuse (1) abdichtet und daß im Deckel (4) ein luftdurchlässiger Bakterienfilter (11) vorhanden ist.

2. Blutentnahme-Vorrichtung nach Anspruch 1, dadurch gekennzeichnet, daß im Inneren des Gehäuses (1) ein Verdrehschutz (12) angeordnet ist, der ein Verdrehen des Kolbens (2) relativ zum Gehäuse (1) verhindert.

3. Blutentnahme-Vorrichtung nach Anspruch 2, dadurch gekennzeichnet, daß der Verdrehschutz eine ebene Scheibe (12) ist, die mit Vorsprüngen (20) in Nuten (19) des Gehäuses drehfest gehalten ist und daß der Verdrehschutz (12) eine Öffnung (27) für den Durchtritt der Kolbenstange (10) aufweist, wobei diese Öffnung zwei einander gegenüberliegende abgeflachte Seiten (28) aufweist und die Kolbenstange (10) ebenfalls zwei entsprechende abgeflachte Seiten (25) aufweist.

4. Blutentnahme-Vorrichtung nach Anspruch 3, dadurch gekennzeichnet, daß die Kolbenstange (10) ein Außengewinde (26) und die Gewindehülse (9) ein Innengewinde (32) aufweist.

5. Blutentnahme-Vorrichtung nach einem der Ansprüche 1 bis 4, dadurch gekennzeichnet, daß die Dichtung zwischen dem Gehäuse (1) und dem Deckel (4) eine Ringdichtung (7) ist, die in einer Nut (8) des Gehäuses (1) gehalten ist und mit der Innenwandung des Deckels (4) in Berührung steht.

6. Blutentnahme-Vorrichtung nach einem der Ansprüche 1 bis 5, dadurch gekennzeichnet, daß der Deckel (4) in Axialrichtung durch einen Schnappverschluß (5,6) am Gehäuse (1) gehalten ist.

7. Blutentnahme-Vorrichtung nach Anspruch 6, dadurch gekennzeichnet, daß der Schnappverschluß durch eine radial vom Gehäuse abstehende ringförmige Schulter (6) und durch am Deckel (4) angebrachte, die Schulter (6) hintergreifende Rastvorsprünge (5) gebildet ist.

8. Blutentnahme-Vorrichtung nach einem der Ansprüche 1 bis 7, dadurch gekennzeichnet, daß am Gehäuse (1) Drehbegrenzungsanschläge (21) für die Begrenzung der Drehung des Deckels (4) vorgesehen sind.

9. Blutentnahme-Vorrichtung nach einem der Ansprüche 1 bis 8, dadurch gekennzeichnet, daß der Verdrehschutz (12) zwischen einer Stirnseite der Gewindehülse (9) und einer Schulter (37) im Inneren des Gehäuses (1) in Axialrichtung fixiert ist.

10. Blutentnahme-Vorrichtung nach einem der Ansprüche 1 bis 9, dadurch gekennzeichnet, daß die Länge der Gewindehülse (9) mindestens der Verschiebelänge des Kolbens (2) entspricht.

11. Blutentnahme-Vorrichtung nach einem der Ansprüche 1 bis 10, dadurch gekennzeichnet, daß das zur Außenseite des Deckels (4) weisende Ende der Gewindehülse (9) durch den Bakterienfilter (11) verschlossen ist.

## Claims

1. Blood sampling device comprising a piston/cylinder arrangement which serves as an intermediate reservoir for diluted blood having an essentially cylindrical housing (1) which communicates with a flow through channel (14) and which is closed by a rotatable cap (4) having the shape of a dome and comprising a piston (2) which is movable inside the housing (1) and which is connected to cap (4) by means of a thread (26) characterized in that the cap (4) comprises a threaded sleeve (9) interacting with thread (26) of piston rod (10) of piston (2), cap (4) comprises a sealing (7) which seals housing (1) and that cap (4) is provided with a bacterial filter (11) which is permeable to air.

2. Blood sampling device according to claim 1, characterized in that inside housing (1) there is provided a protection against rotation (12) which prevents rotation of piston (2) with respect to housing (1).

3. Blood sampling device according to claim 2, characterized in that the protection against rotation is constituted by an even disk (12) being rotationally fixed by protrusions (20) engaging notches (19) of the housing and that the protection against rotation (12) comprises an opening (27) allowing the passage of piston rod (10), this opening comprises two flattened sides (28) opposing each other and piston rod (10) also comprises two flattened sides (25) corresponding thereto.

4. Blood sampling device according to claim 3, characterized in that piston rod (10) comprises an external thread (26) and that threaded sleeve (9) comprises an internal thread (32).

5. Blood sampling device according to one of the claims 1 to 4, characterized in that the sealing between housing (1) and cap (4) is a circumferential joint (7) which is arranged in a notch (8) of housing (1) and which is in contact with the inner wall of cap (4).

6. Blood sampling device according to one of the claims 1 to 5, characterized in that cap (4) is fixed in an axial direction at housing (1) by means of a snap lock (5, 6).

7. Blood sampling device according to claim 6, characterized in that the snap lock is constituted by a shoulder (6) radially projecting from the housing and by snap protrusions (5) provided at cap (4) and extending behind shoulder (6).

8. Blood sampling device according to one of the claims 1 to 7, characterized in that the housing (1) is provided with rotation stops (21) limiting the rotation of cap (4).

9. Blood sampling device according to one of the claims 1 to 8, characterized in that the protection against rotation (12) is fixed in an axial direction between a front side of threaded sleeve (9) and a shoulder (37) inside housing (1).

10. Blood sampling device according to one of the claims 1 to 9 characterized in that the length of threaded sleeve (9) is at least equal to the stroke of piston (2).

11. Blood sampling device according to one of the claims 1 to 10 characterized in that the end of threaded sleeve (9) facing the outside of cap (4) is closed by the bacterial filter (11).

## Revendications

1. Dispositif de prise de sang avec un agencement de piston/cylindre servant de réservoir intermédiaire pour le sang dilué, avec un corps (1) essentiellement cylindrique qui communique avec un canal de passage (14) et qui est fermé par un couvercle tournant (4) en forme de capuchon, et avec un piston (2) déplaçable à l'intérieur du corps (1), ce piston étant raccordé au couvercle (4) par l'intermédiaire d'un filetage (26), caractérisé en ce que le couvercle (4) présente une douille taraudée (9) qui agit en combinaison avec le filetage (26) de la tige de piston (10) du piston (2), que le couvercle (4) comprend un joint (7) qui rend étanche le corps (1) et que le couvercle (4) contient un filtre à bactéries (11) perméable à l'air.

2. Dispositif de prise de sang selon la revendication 1, caractérisé en ce qu'à l'intérieur du corps (1) est disposée une sécurité contre la rotation (12) qui empêche une rotation du piston (2) par rapport au corps (1).

3. Dispositif de prise de sang selon la revendication 2, caractérisé en ce que la sécurité contre la rotation est une rondelle plate (12) qui est maintenue dans des rainures (19) du corps par des saillies (20) d'une façon empêchant la rotation, et en ce que la sécurité contre la rotation (12) présente un orifice (27) pour la pénétration de la tige de piston (10), cet orifice présentant deux faces aplaties opposées (28) et la tige de piston (10) présentant également deux faces aplaties correspondantes (25).

4. Dispositif de prise de sang selon la revendication 3, caractérisé en ce que la tige de piston (10) présente un filetage extérieur (26) et la douille taraudée (9) présente un filetage intérieur (32).

5. Dispositif de prise de sang selon l'une des revendications 1 à 4, caractérisé en ce que le joint entre le corps (1) et le couvercle (4) est un joint annulaire (7) qui est maintenu dans une rainure (8) du corps (1) et est en contact avec la paroi intérieure du couvercle (4).

6. Dispositif de prise de sang selon l'une des revendications 1 à 5, caractérisé en ce que le couvercle (4) est maintenu sur le corps (1) dans la direction axiale par une fermeture à déclic (5,6).

7. Dispositif de prise de sang selon la revendication 6, caractérisé en ce que la fermeture à déclic se compose d'un épaulement annulaire (6) dépassant du corps dans le sens radial et de saillies d'arrêt (5) disposées sur le couvercle (4) et prenant l'épaulement (6) par l'arrière.

8. Dispositif de prise de sang selon l'une des revendications 1 à 7, caractérisé en ce que sur le corps (1) sont prévues des butées de limitation de rotation (21) pour limiter la rotation du couvercle (4).

9. Dispositif de prise de sang selon l'une des revendications 1 à 8, caractérisé en ce que la sécurité contre la rotation (12) est fixée dans la direction axiale à l'intérieur du corps (1) entre une face frontale de la douille taraudée (9) et un épaulement (37).

10. Dispositif de prise de sang selon l'une des revendications 1 à 9, caractérisé en ce que la longueur de la douille taraudée (9) correspond au moins à la longueur de déplacement du piston (2).

11. Dispositif de prise de sang selon l'une des revendications 1 à 10, caractérisé en ce que l'extrémité de la douille taraudée (9) qui donne sur la face externe du couvercle (4) est fermée par le filtre à bactéries (11).
